# EUROPÄISCHE PATENTSCHRIFT

(11) **EP 0 283 555 B1**
(45) Veröffentlichungstag und Bekanntmachung des Hinweises auf die Patenterteilung: **11.08.1993**
(21) Anmeldenummer: 87114094.3
(22) Anmeldetag: 26.09.1987
(51) Int. Cl.: G02B 26/08, G02B 23/02, B23K 26/02, A61B 17/36

(54) **Strahlungsführungsoptik für Laserstrahlung**
Beam guiding optics for laser radiation
Optique à guidage de faisceaux lumineux pour rayonnements de laser

(30) Priorität: 21.03.1987 DE 3709351
(43) Veröffentlichungstag der Anmeldung: 28.09.1988
(73) Patentinhaber: Heraeus Instruments GmbH, 63450 Hanau (DE)
(72) Erfinder: Gorisch, Wolfram, Dr., D-8750 Aschaffenburg (DE)
(74) Vertreter: Kühn, Hans-Christian

(56) Entgegenhaltungen:
- EP-A- 0 046 593
- EP-A- 0 065 785
- EP-A- 0 185 233
- US-A- 3 986 767
- US-A- 4 192 573

## Beschreibung

Die Erfindung betrifft eine Strahlführungsoptik für Laserstrahlung, insbesondere für die Strahlung eines medizinischen Lasers, zur stufenlosen Brennweitenverstellung, mit mindestens einem konkaven Umlenkspiegel und einer auf Teile der Strahlführungsoptik wirkenden Verstelleinrichtung.

Eine Strahlführungsoptik für medizinische Anwendungen ist aus der US-A-4396 285 bekannt. Das dort beschriebene Lasersystem mit einem Arbeitslaser und einem Ziellaser weist einen verschwenkbaren, konkav gewölbten Spiegel auf, mit dem die Lage des Brennpunktes des Laserstrahls in der Arbeitsebene verändert werden kann. Mit dieser Anordnung ist nicht die Möglichkeit gegeben, die Brennweite und damit die Lage des Brennpunktes in Strahlrichtung zu verändern.

Des weiteren sind Linsenoptiken für Lasersysteme, die üblicherweise einen Helium-Neon-Ziellaser und einen CO₂-Arbeitslaser aufweisen, bekannt, bei denen die Brennweite durch Verschieben der Linsen variabel eingestellt werden kann. Solche Optiken, die Linsen enthalten, weisen eine Dispersion auf, so daß infolge der verschiedenen Wellenlängen des Ziellasers und des Arbeitslasers die Brennpunkte von Ziellaser und Arbeitslaser in ihrer räumlichen Lage nicht übereinstimmen. Bei mittig auf die Linsen treffenden Strahlen weichen die Brennpunkte longitudinal, bei außermittigem Strahlverlauf auch lateral voneinander ab; hierdurch verfehlt der Zielstrahl seinen Zweck.

Ausgehend von diesem Stand der Technik liegt der vorliegenden Erfindung die Aufgabe zugrunde, eine Strahlführungsoptik, insbesondere für die Strahlung eines medizinischen Lasers zu schaffen, bei der die Brennweite stufenlos verstellbar ist und Abbildungsfehler, z.B. aufgrund der Dispersion linsenoptische Abbildungssysteme weitgehend vermieden werden.

Diese Aufgabe wird durch die im Anspruch 1 definierte Strahlführungsoptik gelöst.

Wesentlich ist, daß die Laserstrahlen sowohl von einem konkaven und als auch von einem konvexen Spiegel reflektiert werden, bevor sie auf das Arbeitsfeld fallen. Die gegeneinander in ihrem Abstand verstellbar angeordneten Spiegel ermöglichen eine Verschiebung der Fokuslage in Strahlrichtung. Durch die im Strahlengang angeordneten beiden Spiegel mit komplementärer Krümmung werden Bildfehler infolge der Dispersion weitgehend vermieden, da keine optische Brechung stattfindet, wie dies bei Linsenoptiken der Fall wäre. Durch die Spiegel mit komplementärer Krümmung, bei denen es sich um Flächen aus Rotationskörpern von Kegelschnitten handelt, also um Ellipsoide oder Paraboloide, die in mindestens einer der zwei zueinander senkrechten Richtungen anisotrope Krümmungsradien aufweisen, werden Aberrationen vermieden. Die geometrische Form der Ellipsoide bzw. Paraboloide kann mit Hilfe des bekannten mathematischen Formalismus zur Bestimmung von Kegelschnitten berechnet werden, wobei die erforderlichen Brennweiten durch den geometrisch-optischen bzw. gauss-optischen Strahlverlauf vorgegeben werden können.

Sollten geringe Aberrationen toleriert werden, so können anstelle der Rotationskörper von Kegelschnitten auch Toroidspiegel verwendet werden. Beispielsweise sind die Krümmungsradien solcher toroidaler Spiegel mit R/k und, in der dazu senkrechten Richtung, mit R x k zu wählen, wobei k = √2̅ beträgt (bei einem Einfallswinkel von 45°). Durch die Anordnung der Spiegel innerhalb des Schlittens derart, daß die Strahlachsen des in die Verstelleinrichtung einfallenden und ausfallenden Strahles parallel zueinander verlaufen und der Schlitten parallel zu den Strahlachsen, d.h. in Richtung der Strahlachsen, zu verschieben ist, kann in einfacher Weise die Brennweite der Optik und damit die Lage des Brennpunktes eingestellt werden.

Bevorzugt wird in Strahlrichtung gesehen als ausgangsseitger Schlitten-Spiegel der konkav oder konvex gekrümmte Spiegel eingesetzt. Innerhalb der Verstelleinrichtung bzw. des Schlittens kann als eingangsseitiger Schlitten-Spiegel ein Planspiegel verwendet werden.

Je nach der erwünschten Lage des Brennpunktes und des erforderlichen Strahldurchmessers wird auch der eingangsseitige Schlitten-Spiegel als konvexer oder konkaver Spiegel ausgebildet, wobei dann der im Strahlengang außerhalb der Verstelleinrichtung vorgesehene mindestens eine Umlenkspiegel, der den Strahl in Richtung der Arbeitsebene umlenkt, eine dem eingangsseitigen Schlitten-Spiegel entsprechende Krümmung aufweist.

Bevorzugt wird der Umlenkspiegel schwenkbar um mindestens eine Achse senkrecht zur Strahlachse angeordnet, so daß die Anordnung neben der Brennpunkteinstellung auch eine Verschwenkung des Brennpunktes in einer bestimmten Arbeitsebene zuläßt. Durch diese Maßnahme ist der Brennpunkt der Laserstrahlung in zwei weiteren, d.h. insgesamt in drei Raumrichtungen einstellbar.

Die Verstelleinrichtung und der Umlenkspiegel können in einer Baueinheit ausgeführt sein, die dann bevorzugt an einem mehrere Gelenke aufweisenden Strahlführungsarm angeordnet ist, so daß der Brennpunkt mittels einer solchen Strahlführungsoptik in verschiedene, räumlich unterschiedlich orientierte Arbeitsebenen verschwenkbar ist.

In einer konstruktiv einfachen Ausführungsform wird der Schlitten mittels eines Zahnrad-Antriebes linear verschoben, bevorzugt in der Form, daß der Schlitten in einer Nuten-Führung geführt ist und eine Zahnstange aufweist, die über ein darin eingreifendes Zahnrad zusammen mit dem Schlitten verschoben wird. Andere Verschiebemechaniken sind möglich, insbesondere in Form einer Zylinder-Buchsen-Führung.

Weitere Einzelheiten der Erfindung ergeben sich aus der nachfolgenden Beschreibung verschiedener Ausführungsbeispiele anhand der Zeichnung. In der Zeichnung zeigt
- Figur 1: schematisch ein Lasersystem mit einer erfindungsgemäßen Strahlführungsoptik,
- Figur 2: in einer detaillierten Darstellung die Strahlführungsoptik nach Figur 1
- Figur 3: eine Anordnung, bei der gegenüber der Ausführungsform nach Figur 2 der verschiebbare Schlitten und ein Umlenkspiegel gegenüber dem Strahlführungsarm mechanisch entkoppelt sind,
- Figur 4: eine Anordnung, bei der die Verstelleinrichtung in vertikaler Richtung verschiebbar ist,
- Figur 5: eine Anordnung, bei der gegenüber der Ausführungsform nach Figur 4, ein verschwenkbarer Umlenkspiegel eingesetzt und der Schnitten vertikal verschiebbar ist, und
- Figur 6: eine der Ausführungsform nach Figur 5 entsprechende Anordnung, jedoch mit horizontal verschiebbarem Schlitten.

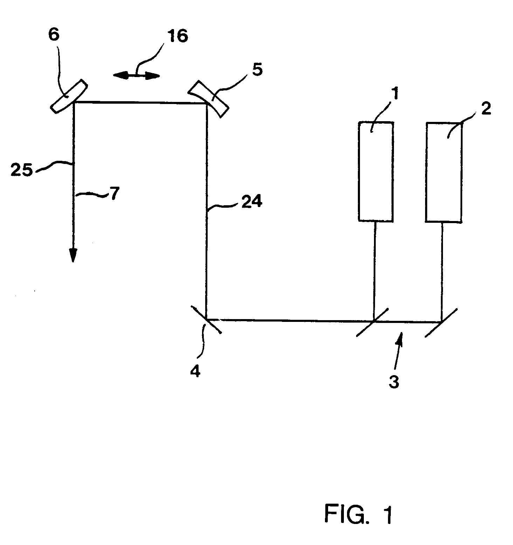

Das in Figur 1 gezeigte Lasersystem weist als Pilot- oder Ziellaser 1 einen Neonlaser und als Arbeitslaser 2 einen CO₂-Laser auf, deren Strahlen über eine Optik 3 zusammengeführt werden, bevor die Strahlen auf einen Umlenkspiegel 4 fallen. Von diesem Umlenkspiegel 4 führen die Strahlen über einen konkaven Spiegel 5 und einen konvexen Umlenkspiegel 6 in Richtung der Arbeitsebene, durch den Pfeil 7 angedeutet.

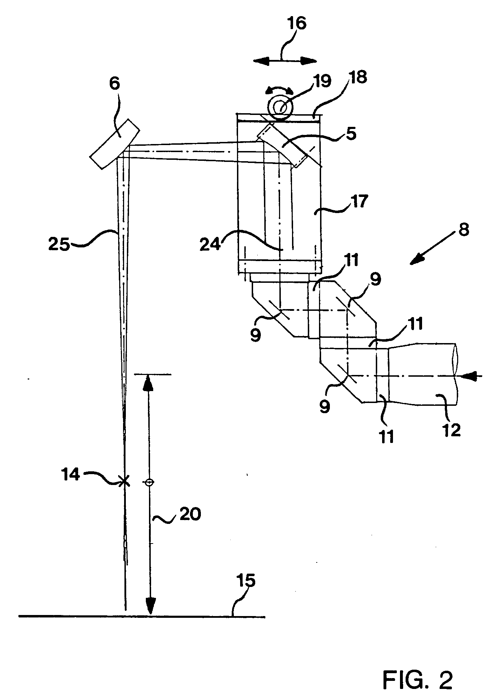

Wie das Ausführungsbeispiel nach Figur 2 zeigt, ist der konkave Spiegel 5, wie er in Figur 1 angedeutet ist, am Ende eines Strahlführungsarm 8 angeordnet. Dieser Strahlführungsarm 8, bei dem es sich um einen gelenkigen Spiegelarm handelt, weist drei Planspiegel 9 in jeweils einem Gelenkteil 10 auf, die den Strahl jeweils um 45° umlenken. Die einzelnen Gelenkteile 10 sind über zwei Gelenke 11 miteinander verbunden; ein weiteres Gelenk 11 ist in Strahlrichtung gesehen zwischen dem ersten Gelenk 11 und einem Arm 12 angeordnet. Durch die drei Gelenke 11, gegebenfalls durch ein zusätzliches Gelenk 13 am Ende des letzten Gelenkteils 10, kann der konkave Spiegel 5 und der Umlenkspiegel 6 in eine beliebige Raumrichtung verschwenkt werden, woraus folgt, daß auch der Umlenkspiegel 6 von dem Strahlführungsarm getragen wird.

Durch die beiden Spiegel 5 und 6, wobei es sich in der Ausführung nach Figur 2 bei dem Umlenkspiegel 6 um einen konvexen Spiegel handelt, werden die parallelen Laserstrahlen in einem gemeinsamen Brennpunkt 14 fokussiert. Um die Lage des Brennpunktes 14 senkrecht zur Arbeitsebene 15 zu verändern, wird die Abstand zwischen dem konvexen Umlenkspiegel 6 und dem konkaven Spiegel 5 verändert. Hierzu ist der Strahlführungsarm 8 horizontal in Richtung des Pfeiles 16 in nicht näher dargestellten Führungen verschiebbar geführt. Um den Abstand der beiden Spiegel stufenlos einstellen zu können, greift in eine Zahnstange 18, die an der oberen Stirnseite des Schlittens 17 befestigt ist, ein Zahnrad 19 ein. Dieser Antrieb kann auch als Schneckengetriebe ausgeführt sein.

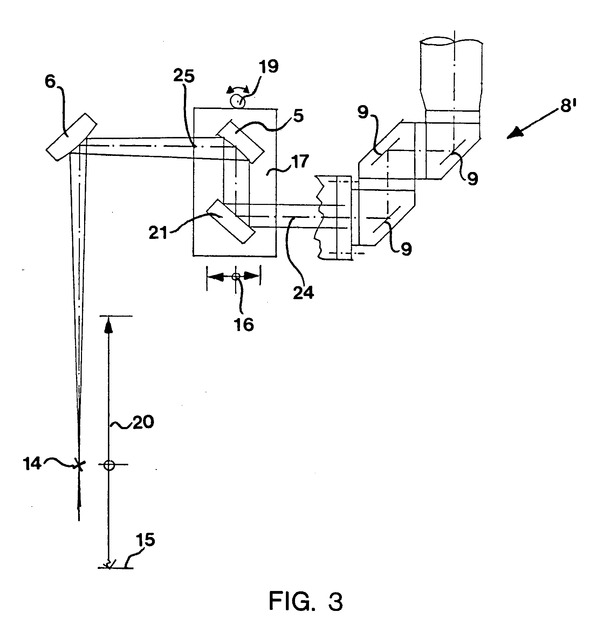

Während bei der Ausführungsform nach Figur 2 zum Verstellen des Brennpunktes 14 - der Verstellbereich des Brennpunktes ist durch die Pfeile 20 angezeigt - der Abstand zwischen dem konvexen Umlenkspiegel 6 und dem konkaven Umlenkspiegel 5 verändert wird, indem der Strahlführungsarm 8 mit dem darin angeordneten konkaven Spiegel 5 relativ zu dem Umlenkspiegel 6 verändert wird, bleiben nach der Ausführungsform nach Figur 3 sowohl der Umlenkspiegel 6 als auch der Strahlführungsarm 8ʹ in ihrem Abstand zueinander fest. Lediglich der Schlitten 17 wird über den Zahnstangen-Zahnrad-Antrieb 18, 19 zwischen dem Umlenkspiegel 6 und dem Strahlführungsarm 8ʹ verschoben. Innerhalb des Schlittens 17 ist, im Gegensatz zu der Ausführungsform nach Figur 2, ein zusätzlicher Planspiegel 21 angeordnet, der den Strahl von dem letzten Planspiegel 9 in Richtung des konkaven Spiegels 5 umlenkt. Da sowohl der Speigel 9 als auch der Spiegel 21 ein Planspiegel ist, wird der Strahl zwischen diesen beiden Spiegeln parallel geführt, so daß die Veränderung des Abstandes keine Einwirkung auf den Strahl hat. Die Verschiebung des Brennpunktes 14 wird allein durch die Veränderung des Abstandes der Spiegel 5 und 6 bewirkt. In einer weiteren, nicht dargestellten Ausführungsform können die Krümmungen der Spiegel 5 und 6 vertauscht werden. Dadurch wird der Strahl 25 aufgeweitet, was zu einem kleineren Fokuspunkt 14 führt, der sich nach gauss-optischen Formeln berechnen läßt.

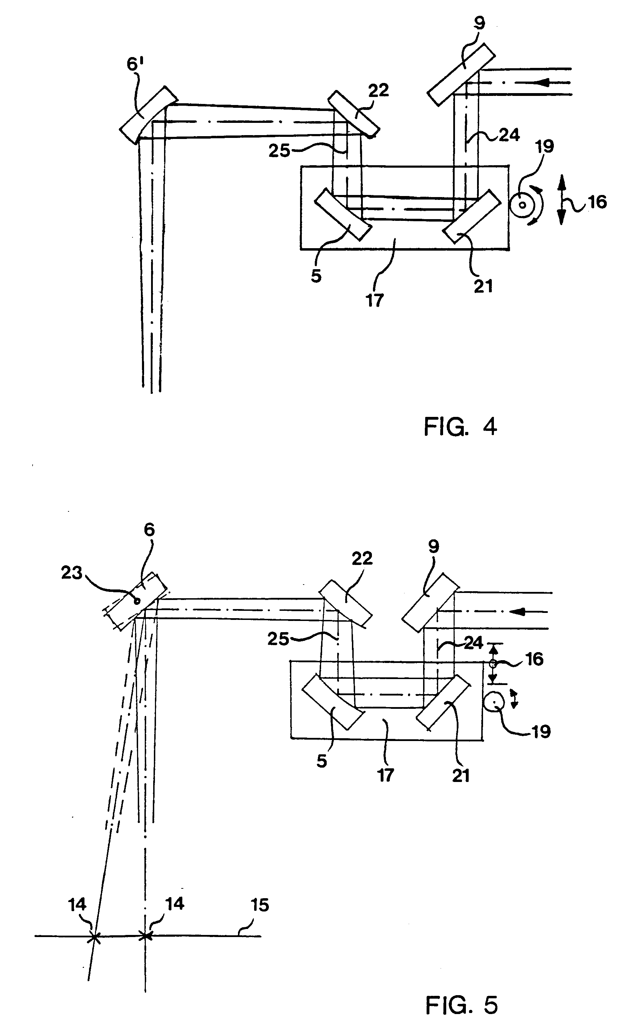

In einer weiteren zur Figur 3 alternativen Ausführungsform ist in der Ausführungsform nach Figur 4 der Schlitten in vertikaler Richtung verschiebbar, der ebenfalls eingangsseitig einer zusätzlichen Planspiegel 21 sowie einen konkaven Spiegel 5 aufweist. Außerhalb des Schlittens 17 ist neben dem Umlenkspiegel 6ʹ, der eine konkave Krümmung aufweist, ein zusätzlicher Umlenkspiegel 22 mit konvexer Krümmung vorgesehen, um den über den Spiegel 5 aus den Schlitten 17 austretenden Strahl auf den Spiegel 6ʹ, der in diesem Beispiel konkav gekrümmt ist, zu werfen. In dem gezeigten Ausführungsbeispiel ist die konvexe Krümmung auf die beiden Spiegel 5 und 6ʹ aufgeteilt. Bei geeigneten Krümmungen der Spiegel 5 und 22 kann der Spiegel 6ʹ auch durch einen Planspiegel ersetzt werden. Der Spiegel 9, der den Strahl auf den zusätzlichen Planspiegel 21 in dem Schlitten 17 wirft, kann der ausgangsseitige Spiegel des Strahlführungsarmes 8ʹ sein.

Um den Spiegel bzw. den Brennpunkt 14 in der Arbeitsebene verschwenken zu können, ist der Spiegel 6ʹ, der in Figur 5 beispielsweise ein Planspiegel ist, um zwei senkrecht zueinander verlaufende Schwenkachsen 23, von denen die eine gezeigt ist, schwenkbar gelagert. In der übrigen Spiegelanordnung entspricht die Ausführung der Figur 5 der Ausführung nach Figur 4.

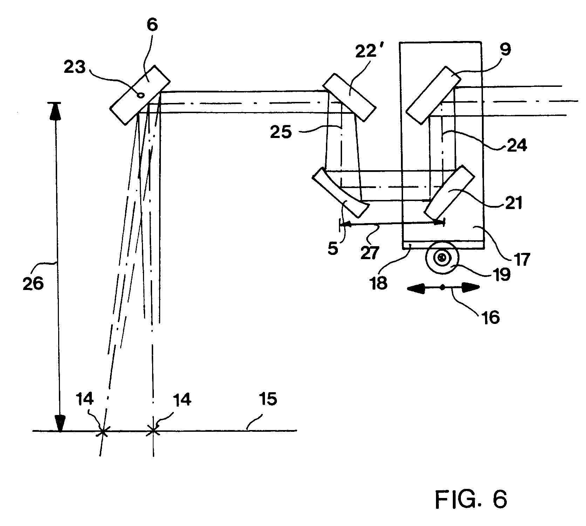

Bei der Ausführung nach Figur 6 wird entsprechend der Ausführung nach Figur 5 ausgangsseitig der Strahlanordnung ein Planspiegel als Schwenk- spiegel verwendet. Falls hier gekrümmte Spiegel verschwenkt werden, trifft der Laserstrahl nicht mit 45°, sondern mit einem variablen Winkel auf den Spiegel auf. Bei Einfallswinkeln, die von 45° abweichen, ergibt sich ein Bild- fehler (Astigmatismus), der die Strahlkaustik im Fokusbereich unzulässig deformiert. Dies tritt nicht bei solchen ebenen Spiegeln auf.

Im Gegensatz zu der Ausführungsform nach Figur 5 ist der Umlenkspiegel 22, in Figur 6 mit 22ʹ bezeichnet, ein Planspiegel. Außerdem wird der Schlitten 17 horizontal verschoben und damit der Abstand zwischen den Spiegeln 5 und 21 verändert. Die Brennweite des Spiegels 21 (Rotations-Paraboloid) nach Figur 6 beträgt -100 mm, die Brennweite des Spiegels 5 (Rotations-Ellipsoid; Abbildungsmaßstab 1:3) liegt bei 90 mm. Der Verschiebebereich 16 des Schlittens 17 beträgt 20 mm, der Abstand der Spiegel 5 und 21, durch die Pfeile 27 angedeutet, beträgt minimal 15 mm und maximal 35 mm; der Fokusabstand, in Figur 6 mit dem Bezugszeichen 26 bezeichnet, liegt demzufolge bei minimal 250 mm und maximal 400 mm.

Wie in den Figuren ersichtlich ist, verlaufen die Verschiebeachsen der Schlitten 17, die durch die Pfeile 16 angedeutet sind, parallel zu den Strahlachsen des auf den Eingangsspiegel des Schlittens treffenden Eingangsstrahls 24 und Ausgangsstrahls 25. Grundsätzlich wird mit dem Verschieben der Schlitten 17 der Abstand zwischen einem konvexen und einem konkaven Spiegel verändert, so daß mit der Abstandsänderung die Fokusebene verändert wird. Die beiden Spiegel mit komplementärer Krümmung, bei denen es sich um Flächenelemente aus Rotationskörpern von Kegelschnitten handelt, also um rotations-ellipsoide oder rotations-paraboloide Flächenelemente, sind derart zueinander ausgerichtet, daß die zugehörigen Brennpunkte des jeweiligen Spiegels in der Ebene liegen, die durch den einfallenden und den reflektierten Strahl aufgespannt wird.

## Patentansprüche

1. Strahlführungsoptik für Laserstrahlung, insbesondere für die Strahlung eines medizinischen Lasers, mit einer Verstelleinrichtung zur stufenlosen Brennweitenverstellung, die einen linear bewegbaren Schlitten (17) aufweist, der mindestens zwei fest zueinander angeordnete Schlitten-Spiegel (5, 9, 21) trägt, von denen mindestens einer konkav oder konvex gekrümmt ist, wobei die Strahlachse des auf den einen Spiegel fallenden Eingangsstrahles (24) parallel zu der Strahlachse des aus der Verstelleinrichtung austretenden Strahles (25) verläuft und wobei die Verschiebeachse (16) des Schlittens (17) parallel zu der Strahlachse des Eingangs- und Ausgangsstrahles (24, 25) ausgerichtet ist, und mit mindestens einem im Strahlengang außerhalb der Verstelleinrichtung, angeordnetten Umlenkspiegel (6, 6ʹ, 22), der eine zur Krümmung des mindestens einen gekrümmten Schlitten-Spiegels (5, 9, 21) komplementäre Krümmung aufweist, wobei die Spiegel mit komplementärer Krümmung, bei denen es sich um Flächenelemente aus Rotationskörpern von Kegelschnitten handelt, derart zueinander ausgerichtet sind, daß die zugehörigen Brennpunkte des jeweiligen Spiegels in der Ebene liegen, die durch einfallenden und reflektierten Strahl des jeweiligen Spiegels aufgespannt wird.

2. Strahlführungsoptik nach Anspruch 1, dadurch gekennzeichnet, daß der in Strahlrichtung gesehen ausgangsseitige Schlitten-Spiegel (5) der konkav oder konvex gekrümmte Spiegel ist.

3. Strahlführungsoptik nach Anspruch 1 oder 2, dadurch gekennzeichnet, daß der in Strahlrichtung gesehen eingangsseitige Schlitten-Spiegel (9, 21) ein Planspiegel ist.

4. Strahlführungsoptik nach Anspruch 2, dadurch gekennzeichnet, daß der ausgangsseitige Schlitten-Spiegel (5) ein konvex gekrümmer Spiegel ist.

5. Strahlführungsoptik nach einem der Ansprüche 1 bis 4, dadurch gekennzeichnet, daß der Umlenkspiegel (6, 6ʹ) in Strahlrichtung gesehen der Verstelleinrichtung nachgeordnet ist.

6. Strahlführungsoptik nach Anspruch 5, dadurch gekennzeichnet, daß der Umlenkspiegel um mindestens eine Achse (23) senkrecht zur Achse des einfallenden Strahls schwenkbar angeordnet ist.

7. Strahlführungsoptik nach einem der Ansprüche 1 bis 6, dadurch gekennzeichnet, daß der Schlitten (17) und der Umlenkspiegel (6, 6ʹ) eine Baueinheit bilden.

8. Strahlführungsoptik nach Anspruch 7, dadurch gekennzeichnet, daß die den Schlitten (17) und den Umlenkspiegel (6, 6ʹ) aufweisende Baueinheit an einem mehrere Gelenke aufweisenden Strahlführungsarm (8, 8ʹ) angeordnet ist.

9. Strahlführungsoptik nach einem der Ansprüche 1 bis 8, dadurch gekennzeichnet, daß der Schlitten (17) mittels eines Zahnrad-Antriebes (18, 19) linear verschiebbar ist.

10. Strahlführungsoptik nach Anspruch 9, dadurch gekennzeichnet, daß der Schlitten (17) in der Verstelleinrichtung mittels einer Nuten-Führung geführt ist und eine Zahnstange (18) aufweist, in die ein mit einem Verstellelement verbundenes Zahnrad (19) eingreift.

## Claims

1. Optical beam guide for Laser beam radiation, particularly the radiation of a medical laser, with an adjusting device, for infinitely variable focal length adjustment, which has a linearly moveable carriage (17) which carries at least two carriage mirrors (5, 9, 21), which are arranged fixed relative each other, of which at least one is curved in a concave or convex shape, in which the beam axis of the incoming beam (24) striking one of the mirrors extends parallel to the beam axis of the beam (25) emanating from the adjusting device and in which the displacement axis (16) of the carriage (17) is aligned parallel to the beam axis of the incoming and outgoing beams (24, 25), and with at least one deflection mirror (6, 6', 22), arranged in the beam passage outside the adjusting device, which has a complementary curve for curving of at least one carriage mirror (5, 9, 21), in which the mirrors with the complementary curve, relating to surface elements of rotational bodies of conical sections, are aligned with respect to each other in such a way that the associated focal points of the respective mirror are situated in the plane which is stressed by the incident and reflecting beam of the respective mirror.

2. Optical beam guide according to claim 1, **characterised** the carriage mirror (5) on the output side, when viewed in the beam direction, is the concave or convex shaped mirror.

3. Optical beam guide according to claim 1 or 2, **characterised** in that the carriage mirror (9, 21) on the input side, when viewed in the beam direction, is a plane mirror.

4. Optical beam guide according to claim 2, **characterised** in that the carriage mirror (5) on the output side is a convex shaped mirror.

5. Optical beam guide according to one of the claims 1 to 4, **characterised** in that when viewed in the beam direction the deflection mirror (6, 6') is arranged after the adjusting device.

6. Optical beam guide according to claim 5, **characterised** in that the deflection mirror is arranged pivotally about at least one axis (23) perpendicular to the axis of the incident beam.

7. Optical beam guide according to one of the claims 1 to 6, **characterised** in that the carriage (17) and the deflection mirror (6, 6') form one unit.

8. Optical beam guide according to claim 7, **characterised** in that the unit comprising the carriage (17) and deflection mirror (6, 6') is arranged on a beam guide arm (8, 8') having a number of joints.

9. Optical beam guide according to one of the claims 1 to 8, **characterised** in that the carriage (17) is linearly displaceable by means of a gear drive (18, 19).

10. Optical beam guide according to claim 9, **characterised** in that the carriage (17) is guided in the adjusting device by means of a channel guide and has a gear rack (18) in which engages a gear (19) connected to an adjusting element.

## Revendications

1. Optique de guidage des faisceaux lumineux pour rayonnement laser, en particulier pour le rayonnement d'un laser médical, comportant un dispositif de réglage qui est prévu pour le réglage continu de la distance focale et qui présente un chariot (17) qui peut se déplacer linéairement et qui porte au moins deux miroirs de chariot (5, 9, 21) disposés fixes l'un par rapport à l'autre, dont au moins l'un est à courbure concave ou convexe, étant précisé que l'axe du faisceau (24) qui tombe sur le premier miroir est orienté parallèlement à l'axe du faisceau (25) qui sort hors du dispositif de réglage et étant précisé que l'axe de déplacement (16) du chariot (17) est orienté parallèlement à l'axe du faisceau d'entrée et à l'axe du faisceau de sortie (24, 25), et comportant au moins un miroir de changement de direction (6, 6', 22) qui est disposé, sur le chemin des faisceaux, en dehors du dispositif de réglage et qui présente une courbure complémentaire de la courbure du miroir courbe, dont il y a au moins un, (5, 9, 21) du chariot, étant précisé que les miroirs à courbure complémentaire, dans le cas desquels il s'agit d'éléments de surface de corps obtenus par la révolution de coniques, sont orientés l'un par rapport à l'autre de façon telle que les foyers correspondants de chaque miroir se situent dans le plan qui passe par le faisceau incident et le faisceau réfléchi de chaque miroir.

2. Optique de guidage du faisceau lumineux selon la revendication 1, caractérisée par le fait que c'est le miroir de chariot (5) situé du côté de la sortie, vu dans la direction de faisceau lumineux, qui est le miroir à courbure concave ou convexe.

3. Optique de guidage des faisceaux lumineux selon la revendication 1 ou 2, caractérisée par le fait que le miroir sur chariot (9, 21), situé du côté de l'entrée, vu selon la direction du faisceau lumineux, est un miroir plan.

4. Optique de guidage des faisceaux lumineux selon la revendication 2, caractérisée par le fait que le miroir sur chariot (5) situé du côté de la sortie est un miroir à courbure convexe.

5. Optique de guidage des faisceaux lumineux selon l'une des revendications 1 à 4, caractérisée par le fait que le miroir de changement de direction (6, 6') est disposé après le dispositif de réglage, vu selon la direction du faisceau lumineux.

6. Optique de guidage de faisceau lumineux selon la revendication 5, caractérisé par le fait que le miroir de changement de direction est disposé pivotant autour d'au moins un axe (23) perpendiculaire à l'axe du faisceaux incident.

7. Optique de guidage des faisceaux lumineux selon l'une des revendications 1 à 6, caractérisée par le fait que le chariot (17) et le miroir de changement de direction (6, 6') forment un ensemble.

8. Optique de guidage des faisceaux lumineux selon la revendication 7, caractérisée par le fait que l'ensemble présentant le chariot (17) et le miroir de changement de direction (6, 6') est disposé sur un bras (8, 8') de guidage du faisceau lumineux présentant plusieurs articulations.

9. Optique de guidage des faisceaux lumineux selon l'une des revendications 1 à 8, caractérisée par le fait que le chariot (17) peut se déplacer linéairement au moyen d'un mécanisme d'entrainement à pignon (18, 19).

10. Optique de guidage des faisceaux lumineux selon la revendication 9, caractérisée par le fait que le chariot (17) est guidé dans le mécanisme de déplacement au moyen d'un guidage à rainures et présente une crémaillère (18) dans laquelle engrène un pigonon denté (19) lié à un élément de déplacement.
